(19) Europäisches Patentamt
European Patent Office
Office européen des brevets

(11) Veröffentlichungsnummer : **0 473 547 A1**

(12) **EUROPÄISCHE PATENTANMELDUNG**

(21) Anmeldenummer : **91810657.6**

(22) Anmeldetag : **20.08.91**

(51) Int. Cl.⁵ : **C07C 381/12,** G03F 7/027

(30) Priorität : **27.08.90 CH 2765/90**

(43) Veröffentlichungstag der Anmeldung : **04.03.92 Patentblatt 92/10**

(84) Benannte Vertragsstaaten : **AT BE CH DE FR GB IT LI NL SE**

(71) Anmelder : **CIBA-GEIGY AG**
**Klybeckstrasse 141**
**CH-4002 Basel (CH)**

(72) Erfinder : **Steinmann, Alfred, Dr.**
**Les Russilles**
**CH-1724 Praroman (CH)**
Erfinder : **Schädeli, Ulrich, Dr.**
**Route des Coteaux 22**
**CH-1763 Granges-Paccot (CH)**

(54) **Olefinisch ungesättigte Oniumsalze.**

(57) Olefinisch ungesättigte Oniumsalze der Formeln I und II

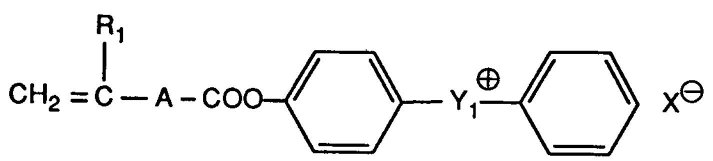

(I)

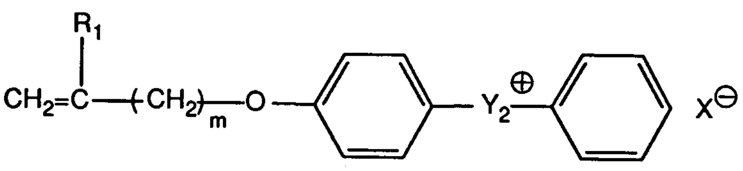

(II),

worin $R_1$ ein Wasserstoffatom oder Methyl und A eine direkte Bindung, Phenylen oder einen der folgenden Reste

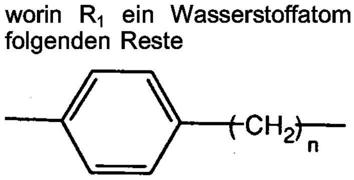 oder $-(CH_2)_o-$

bedeuten, wobei n und o unabhängig voneinander je für eine Zahl von 1 bis 10 stehen, $Y_1$ für J oder S-$R_2$ und $Y_2$ für S-$R_2$ stehen, wobei $R_2$ ein $C_1$-$C_4$-Alkyl, unsubstituiertes oder $C_1$-$C_4$-alkylsubstituiertes Phenyl bedeutet, m für Null oder eine Zahl von 1 bis 10 und X für $BF_4$, $PF_6$, $AsF_6$, $SbF_6$, $SbCl_6$, $FeCl_4$, $SnF_6$, $BiF_6$, $CF_3SO_3$ oder $SbF_5OH$ stehen, lassen sich mit einem olefinisch ungesättigten, eine säurelabile Gruppe enthaltenden Comonomer copolymerisieren und ergeben strahlungsempfindliche Copolymere, die direkt als Photoresists eingesetzt werden können.

EP 0 473 547 A1

Die vorliegende Erfindung betrifft olefinisch ungesättigte Oniumsalze, Verfahren zu deren Herstellung, strahlungsempfindliche Copolymere aus den neuen olefinisch ungesättigten Oniumsalzen und copolymerisierbaren olefinisch ungesättigten Monomeren mit einer säurelabilen Gruppe im Molekül sowie die aus den strahlungsempfindlichen Copolymeren hergestellten Erzeugnisse.

Bei der Herstellung von beispielsweise lithographischen Filmen werden Oniumsalze bekanntlich als niedermolekulare Verbindungen dem Resistharz zugegeben, indem man dieses zusammen mit dem Oniumsalz in einem geeigneten polaren Lösungsmittel löst. Dabei ist man in der Wahl des Lösungsmittels relativ stark eingeschränkt, da die Oniumsalze normalerweise nur in stark polaren Lösungsmitteln, die Resistharze dagegen normalerweise nur in schwach polaren Lösungsmitteln löslich sind. Beim Abdampfen des Lösungsmittels ist das Oniumsalz zwar in der Resistharzmatrix gelöst, doch die Gefahr der Bildung von Klumpen (clusters) und damit das Auskristallisieren des Oniumsalzes aus der Harzmatrix ist relativ gross, insbesondere wenn grössere Mengen an Oniumsalz benötigt werden oder wenn relativ apolare Resistharze verwendet werden, wie in Proceedings of the ACS Division of Polymeric Materials Science and Engineering, Vol. 61, 1989, Seite 185-188 näher beschrieben wird.

Aus der DE-OS 39 02 114 sind strahlungsempfindliche, ethylenisch ungesättigte, copolymerisierbare Sulfoniumsalze bekannt, in denen die reaktive Doppelbindung über einen sogenannten Spacer (Ankergruppe), der unter anderem auch -OCO-Gruppierungen enthalten kann, mit dem Photoinitiator (Sulfoniumsalzgruppe) verbunden ist. Die aus solchen Sulfoniumsalzen durch Copolymerisation mit ungesättigten, eine säurelabile Gruppe aufweisenden Verbindungen erhaltenen Copolymeren sind als Photoresist noch wenig lichtempfindlich, so dass zur Herstellung von Abbildungen der Photoresist mit relativ grossen Dosen bestrahlt werden muss. Ausserdem müssen im Copolymer relativ grosse Mengen an den copolymerisierten Sulfoniumsalzen enthalten sein.

Es wurde nun gefunden, dass sich die oben genannten Nachteile weitgehend vermeiden lassen, wenn man olefinisch ungesättigte Oniumsalze, die einen chemisch anders strukturierten Spacer aufweisen, mit copolymerisierbaren, eine säurelabile Gruppe enthaltenden Verbindungen polymerisiert. Die dabei erhaltenen strahlungsempfindlichen Copolymere weisen als Photoresist ein besseres Lösungsmittelverhalten und ein besseres lithographisches Verhalten auf. Die neuen Copolymeren sind bei geringeren Anteilen an einpolymerisierten Sulfoniumsalzgruppierungen lichtempfindlicher.

Gegenstand vorliegender Erfindung sind olefinisch ungesättigte Oniumsalze der Formeln I und II

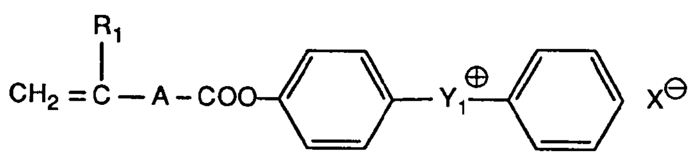

(I)

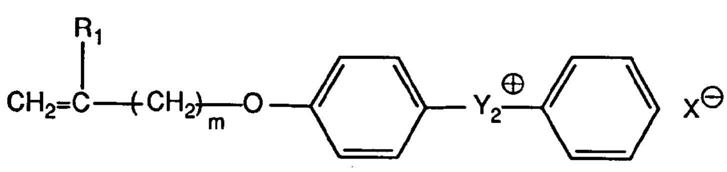

(II),

worin $R_1$ ein Wasserstoffatom oder Methyl und A eine direkte Bindung, Phenylen oder einen der folgenden Reste

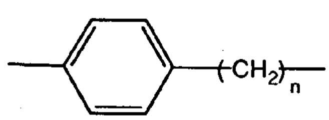 oder 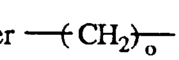

bedeuten, wobei n und o unabhängig voneinander je für eine Zahl von 1 bis 10 stehen, $Y_1$ für -J- oder

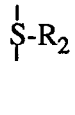

und $Y_2$ für

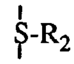

stehen, wobei $R_2$ eine $C_1$-$C_4$-Alkyl, unsubstituiertes oder $C_1$-$C_4$-alkylsubsbtuiertes Phenyl bedeutet, m für Null oder eine Zahl von 1 bis 10 und X für $BF_4$, $PF_6$, $AsF_6$, $SbF_6$, $SbCl_6$, $FeCl_4$, $SnF_6$, $BiF_6$, $CF_3SO_3$ oder $SbF_5OH$ stehen.

Vorzugsweise bedeuten in den Formeln I und II $R_1$ ein Wasserstoffatom oder Methyl und A eine direkte Bindung, Phenylen oder Benzylen bedeuten, stehen $Y_1$ und $Y_2$ je für

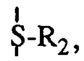

wobei $R_2$ ein unsubstituiertes oder methylsubstituiertes Phenyl bedeutet, und stehen m für Null oder eine Zahl von 1 bis 4 und X für $AsF_6$, $SbF_6$ oder $CF_3SO_3$.

Insbesondere bedeuten in den Formeln I und II $R_1$ ein Wasserstoffatom und A eine direkte Bindung und stehen $Y_1$ und $Y_2$ je für

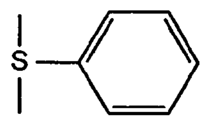

m für Null und X für $AsF_6$, $SbF_6$ oder $CF_3SO_3$.

Bedeutet $R_2$ ein Alkyl oder alkylsubsbtuiertes Phenyl, so können die Alkyle verzweigt oder geradkettig sein. Beispiele dafür sind Methyl, Ethyl, n-Propyl, Isobutyl und n-Butyl.

Besonders bevorzugte Verbindungen der Formel I sind 4-Acryloyloxyphenyldiphenylsulfoniumhexafluoroarsenat, 4-Acryloyloxyphenyldiphenylsulfoniumhexafluoroantimonat und 2-Acryloyloxyphenyldiphenylsulfoniumtriflat.

Die erfindungsgemässen Verbindungen der Formeln I und II können beispielsweise hergestellt werden, indem man 1 Mol eines hydroxysubstituierten Oniumsalzes der Formel III

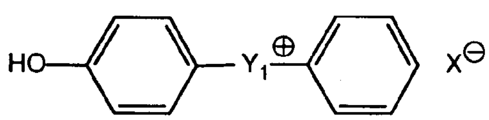

(III),

worin $Y_1$ und X die gleiche Bedeutung wie in Formel I haben, mit 1 Mol eines Säurehalogenids der Formel IV

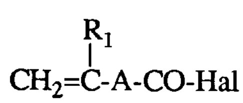

(IV),

worin $R_1$ und A die gleiche Bedeutung wie in Formel I haben und Hal für ein Chlor-, Brom- oder Jodatom steht, in Gegenwart eines tertiären Amins zu Verbindungen der Formel I umsetzt oder, indem man 1 Mol eines hydroxysubstituierten Oniumsalzes der Formel V

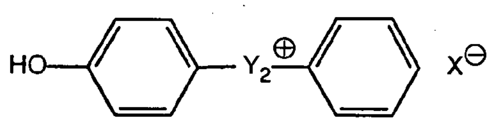

(V),

worin $Y_2$ und X die gleiche Bedeutung wie in Formel II haben, mit 1 Mol einer Halogenverbindung der Formel VI

$$CH_2=CH-(CH_2)_m-Hal \qquad (VI),$$

worin m die gleiche Bedeutung wie in Formel II hat und Hal für ein Chlor-, Brom- oder Jodatom steht, in Gegenwart eines tertiären Amins zu Verbindungen der Formel II umsetzt.

Hydroxysubstituierte Jodoniumsalze der Formel III sind bekannt und werden beispielsweise von F.M. Beringer und I. Lillieu im J. Am. Chem. Soc. 82 (1960), Seite 725 ff beschrieben.

Die hydroxysubstituierten Sulfoniumsalze der Formeln III und V sind in der Literatur noch nicht beschrieben worden und können beispielsweise hergestellt werden, indem man 4-Bromphenol mit Thiophenol-Kupfersalz in molaren Mengen zum 4-Hydroxydiphenylsulfid umsetzt, das anschliessend mit molaren Mengen eines Diphenyljodoniumsalzes, beispielsweise Diphenyljodoniumhexafluoroarsenat, in Gegenwart eines Katalysators, beispielsweise Kupfer(II)benzoat, zum 4-Hydroxyphenyldiphenylsulfoniumsalz umgesetzt wird.

Die Verbindungen der Formel IV stellen bekannte Verbindungen dar, die zum Teil im Handel erhältlich sind. Beispiele für Verbindungen der Formel IV sind Acrylsäurechlorid, Methacrylsäurechlorid, 4-Vinylbenzoesäurechlorid, 4-Isopropenylbenzoesäurechlorid, 4-Vinylphenylessigsäurechlorid, 4-Isopropenylphenylessigsäurechlorid, 4-Vinylphenylpropionsäure und 4-Vinylphenyl-n-buttersäure.

Die Verbindungen der Formel VI stellen ebenfalls bekannte, und zum Teil im Handel erhältliche Verbindungen dar, wie beispielsweise Vinylchlorid und -bromid, Allylchlorid und -bromid und Buten-1-ylchlorid und -bromid.

Bei der Herstellung der erfindungsgemässen Verbindungen können als tertiäre Amine beispielsweise Triethylamin, Benzyldimethylamin, Tributylamin, Pyridin, N,N'-Dimethylpiperazin oder N-Methylpyrrolidon verwendet werden.

Die Umsetzungsreaktion wird vorzugsweise in einem gebräuchlichen organischen inerten Lösungsmittel, wie beispielsweise Methylenchlorid, und bei Raumtemperatur oder leicht erhöhter Raumtemperatur durchgeführt.

Wie eingangs erwähnt, können die neuen olefinisch ungesättigten Oniumsalze mit copolymerisierbaren, eine säurelabile Gruppe enthaltenden Verbindungen polymerisiert werden, wobei strahlungsempfindliche Copolymere erhalten werden, die direkt als Photoresists eingesetzt werden können.

Gegenstand vorliegender Erfindung sind somit auch strahlungsempfindliche Copolymere mit einem Molekulargewicht (Mw) von $10^3$ bis $10^6$, gemessen mittels Gelpermeationschromatographie, enthaltend, bezogen auf die Summe der im Copolymer aus (a) und (b) vorhandenen Strukturelemente,

(a) 1 bis 20 Mol% des wiederkehrenden Strukturelementes der Formel VII oder VIII

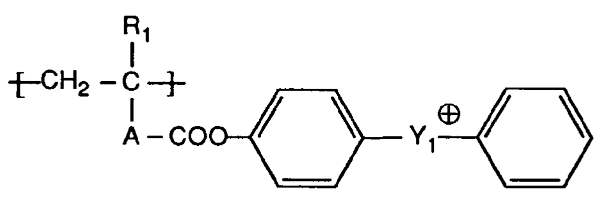

(VII)

oder

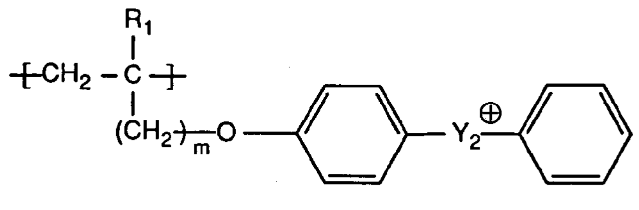

(VIII),

worin $R_1$, A, $Y_1$, $Y_2$ und m die gleiche Bedeutung wie in Formel I bzw. II haben,

(b) 80 bis 99 Mol% eines wiederkehrenden Strukturelementes der Formel IX

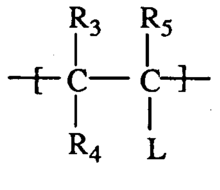

(IX),

worin $R_3$ und $R_4$ unabhängig voneinander je ein Wasserstoffatom, $C_1$-$C_4$-Alkyl oder Phenyl, $R_5$ ein Wasserstoffatom, Methyl oder ein Halogenatom und L einen eine säurelabile Gruppe enthaltenen Rest bedeuten, und

(c) 0 bis 50 Mol% eines wiederkehrenden Strukturelementes der Formel X

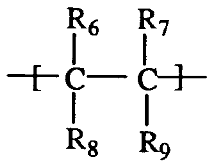

(X),

worin $R_6$ und $R_7$ unabhängig voneinander je ein Wasserstoffatom, unsubstituiertes oder durch Halogenatome, Cyano- oder Nitrogruppen substituiertes $C_1$-$C_4$-Alkyl oder ein unsubstituiertes oder durch Halogenatome, $C_1$-$C_4$-Alkoxy-, Hydroxy-, Cyano- oder Nitrogruppen substituiertes Phenyl oder Naphthyl bedeuten, und $R_8$ und $R_9$ unabhängig voneinander je ein Wasserstoff- oder Halogenatom, unsubstituiertes oder durch Halogenatome, Cyano- oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste $-OR_{10}$, $-COOR_{11}$ oder $-COR_{12}$ bedeuten, wobei $R_{10}$ und $R_{11}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-$C_4$-Alkyl oder $C_1$-$C_4$-Alkoxy substituiertes Phenyl oder Naphthyl bedeuten und $R_{12}$ die gleiche Bedeutung wie $R_{10}$ hat und ausserdem für den Rest

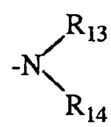

steht, worin $R_{13}$ und $R_{14}$ unabhängig voneinander die gleiche Bedeutung wie $R_{10}$ haben, oder worin $R_8$ und $R_9$, wenn $R_5$ und $R_7$ für ein Wasserstoffatom stehen, zusammen für den Rest

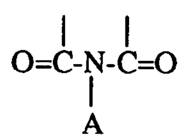

stehen, worin A ein Wasserstoffatom, ein unsubstituiertes oder hydroxysubstituiertes Phenyl oder Benzyl bedeutet, wobei die Summe der Mol% aus (a) und (b) 100 Mol% beträgt und die Mol% von (c) sich auf diese Summe beziehen.

Vorzugsweise enthalten die erfindungsgemässen Copolymeren (a) 5 bis 20 Mol% des wiederkehrenden Strukturelementes der Formel VII oder VIII, (b) 80 bis 95 Mol% des wiederkehrenden Strukturelementes der Formel IX und (c) 0 bis 30 Mol% eines wiederkehrenden Strukturelementes der Formel X.

Die erfindungsgemässen Copolymeren weisen vorzugsweise ein Molekulargewicht (Mw) von 5000 bis 500'000, insbesondere von 20'000 bis 150'000, auf.

In den wiederkehrenden Strukturelementen der Formeln VII und VIII haben $R_1$, A, $Y_1$, $Y_2$ und m die gleiche bevorzugte Bedeutung wie in den Formeln I und II.

Vorzugsweise enthalten die erfindungsgemässen Copolymeren als (a) das wiederkehrende Strukturelement der Formel VII, worin $R_1$ ein Wasserstoffatom und A eine direkte Bindung bedeuten, $Y_1$ und $Y_2$ je für

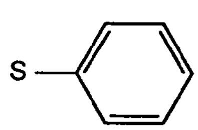

stehen, m Null bedeutet und X für $AsF_6$, $SbF_6$ oder $CF_3SO_3$ steht.

In einer bevorzugten Ausführungsform enthalten die erfindungsgemässen Copolymeren als (b) das wiederkehrende Strukturelement der Formel IX, worin L für einen Rest der Formel XI steht

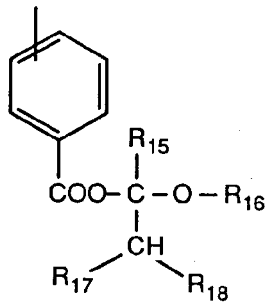

(XI),

worin $R_{15}$ ein Wasserstoffatom oder Methyl, $R_{16}$ ein $C_1$-$C_4$-Alkyl oder $C_6$-$C_{12}$-Aryl und $R_{17}$ und $R_{18}$ unabhängig voneinander je ein Wasserstoffatom, ein $C_1$-$C_4$-Alkyl oder $C_6$-$C_{12}$-Aryl bedeuten, wobei $R_{16}$ und $R_{18}$ zusammen auch einen unsubstituierten oder einen $C_1$-$C_4$-alkyl-, $C_1$-$C_4$-alkoxy-, $C_6$-$C_{12}$-aryl- oder $C_6$-$C_{12}$-aryloxysubstituierten Ethylen-, Propylen- oder Butylenrest bedeuten können.

Die erfindungsgemässen Copolymeren enthalten ferner vorzugsweise als (b) das wiederkehrende Strukturelement der Formel IX, worin L für einen Rest der Formel XII oder XIII steht

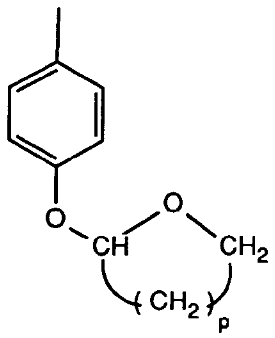

(XII)

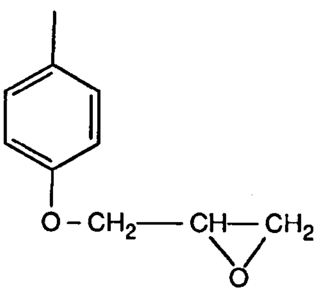

(XIII),

worin p für 2, 3 oder 4 steht.

In einer weiteren bevorzugten Ausführungsform enthalten die erfindungsgemässen Copolymeren als (b) das wiederkehrende Strukturelement der Formel IX, worin L für einen Rest der Formel XIV steht

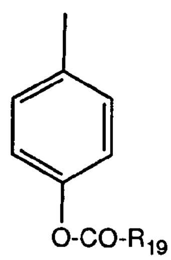

(XIV),

worin $R_{19}$ für $C_1$-$C_4$-Alkyl, $C_1$-$C_4$-Alkoxy oder Oxyalkylen-Si-$(CH_3)_3$ steht.

Bedeuten $R_6$, $R_7$, $R_8$ und $R_9$ im wiederkehrenden Strukturelement der Formel X ein Alkyl, so handelt es sich dabei um geradkettige oder verzweigtkettige, bevorzugt um geradkettige Alkylreste.

Halogenatome als Substituenten können Fluor, Chlor, Brom oder Jod sein und sind bevorzugt Chlor oder Brom.

Als gegebenenfalls substituiertes Alkyl kommen beispielsweise Methyl, Ethyl, 2-Chlorethyl, 2-Nitroethyl, n-Propyl, Isopropyl, n-Butyl, sek.-Butyl, tert.-Butyl, n-Pentyl, Isoamyl, n-Hexyl, 2-Ethylhexyl, n-Decyl, 6-Nitrohexyl oder 9-Bromnonyl in Frage.

Beispiele für substituiertes Phenyl oder Naphthyl sind o-, m- oder p-Chlorphenyl, o-, m-oder p-Tolyl, Xylyl, 2-Nitrophenyl oder α-Chlornaphthyl.

Im wiederkehrenden Strukturelement der Formel X bedeuten $R_6$, $R_7$ und $R_8$ unabhängig voneinander vorzugsweise ein Wasserstoffatom, $C_1$-$C_6$-Alkyl oder Phenyl und bedeutet $R_9$ vorzugsweise ein Halogenatom,

Phenyl oder Benzyl oder einen der Reste $-OR_{10}$, $-COOR_{11}$ oder $-COR_{12}$, worin $R_{10}$, $R_{11}$ und $R_{12}$ unabhängig voneinander je ein Wasserstoffatom, $C_1$-$C_6$-Alkyl oder Phenyl bedeuten und $R_{12}$ ausserdem für den Rest

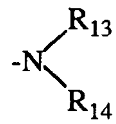

steht, worin $R_{13}$ und $R_{14}$ unabhängig voneinander die bevorzugte Bedeutung von $R_{10}$ haben.

Die erfindungsgemässen Copolymeren können hergestellt werden, indem man

(a) eine Verbindung der Formel I oder II mit

(b) einer Verbindung der Formel IXa

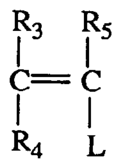

(IXa),

worin $R_3$, $R_4$, $R_5$ und L die gleiche Bedeutung wie in Formel IX haben, im Molverhältnis von a:b von 1:4 bis 1:99 oder mit

(c) einem Gemisch aus einer Verbindung der Formel IXa und einer darin bis zu 50 Mol%, vorzugsweise bis zu 30 Mol%, bezogen auf die Summe der im Molverhältnis von 1:4 bis 1:99 vorliegenden Mole aus (a) und (b), enthaltenen Verbindung der Formel Xa

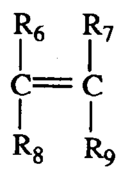

(Xa),

worin $R_6$, $R_7$, $R_8$ und $R_9$ die gleiche Bedeutung wie in Formel X haben, in bekannter Weise radikalisch copolymerisiert.

Die radikalische Copolymerisation kann unter Anwendung verschiedener Techniken durchgeführt werden. Diese sind beispielsweise von S. Sandler und W. Karo in "Polymer Synthesis" Vol. 1-3, 1968, Academic Press, New York, beschrieben worden. Uebliche Polymerisationsverfahren sind beispielsweise Polymerisation in der Masse oder in Lösungsmitteln, Emulsions-, Suspensions- oder Fällungspolymerisation.

Verbindungen, die der Formel IXa entsprechen, sind bekannt. Beispiele für solche Verbindungen sind beispielweise die entsprechenden Acrylat- bzw. Methacrylatester, wie 4-Tetrahydropyranoylphenylmethacrylat, die Styrolderivate, wie 4-tert.-Butoxycarbonyloxystyrol, 4-Acetoxystyrol oder 4-tert.-Butoxystyrol, und die Maleinimidderivate, wie 4-tert.-Butoxycarbonyloxyphenylmaleinimid. Solche Verbindungen sind beschrieben im US-Patent 4,491,628; US-Patent 4,603,101; EP-A-307 356; J. Am. Chem. Soc. 72 (1950), 1200-1202, Polym. Mat. Sci. Eng. (1986), 55, 608-610 und im Polym. Mat. Sci. Eng. (1989), 61, 417ff.

Die Verbindungen der Formel Xa sind bekannt und zum Teil im Handel erhältlich. Neben Olefinen, wie beispielsweise Ethylen oder Propylen, sind als Beispiele für Verbindungen der Formel Xa insbesondere die Vinylverbindungen zu nennen. Beispiele solcher Monomere sind die Styroltypen, wie beispielsweise Styrol, α-Methylstyrol, p-Methylstyrol, p-Hydroxystyrol, p-Acetylstyrol oder p-Hydroxyphenylstyrol, α,β-ungesättigte Säuren, deren Ester oder Amide, wie beispielsweise Acrylsäure, Acrylsäuremethylester, Acrylsäureamid, die entsprechenden Methacrylverbindungen, Maleinsäure, Maleinsäuremethylester, Maleinimid, p-Hydroxyphenylmaleinimide oder 4-Vinylbenzoesäuretert.-butylester, halogenhaltige Vinylverbindungen, wie beispielsweise Vinylchlorid, Vinylfluorid, Vinylidenchlorid oder Vinylidenfluorid, Vinylester, wie beispielsweise Vinylacetat oder Vinylether, wie beispielsweise Methylvinylether oder Butylvinylether.

Weitere geeignete Verbindungen sind beispielsweise die Allylverbindungen, wie Allylchlorid, Allylbromid oder Allylcyanid.

Die Polymerisation wird in der Regel durch einen der üblichen Initiatoren der radikalischen Polymerisation eingeleitet. Dazu zählen thermische Initiatoren, wie Azoverbindungen, beispielsweise Azoisobutyronitril

(AIBN), oder Peroxide, beispielsweise Benzoylperoxid, oder Redoxinitiatorsysteme, wie eine Mischung von Eisen(III)-acetyl-acetonat, Benzoin und Benzoylperoxid, oder photochemische Radikalbildner, wie Benzoin oder Benzilmethylketal.

Die Polymerisation wird bevorzugt in Lösung durchgeführt. Die Reaktionstemperatur bewegt sich im allgemeinen im Bereich von 10 bis 200°C, vorzugsweise zwischen 40 und 150°C, besonders bevorzugt zwischen 40 und 100°C.

Gegebenenfalls anwesende Lösungsmittel müssen unter den Reaktionsbedingungen inert sein. Als Lösungsmittel kommen u.a. aromatische Kohlenwasserstoffe, chlorierte Kohlenwasserstoffe, Ketone und Ether in Betracht. Beispiele dafür sind Benzol, Toluol, Xylol, Ethylbenzol, Isopropylbenzol, Ethylenchlorid, Propylenchlorid, Methylenchlorid, Chloroform, Methylethylketon, Aceton, Cyclohexanon, Diethylether oder Tetrahydrofuran.

Die erfindungsgemässen Copolymeren stellen wertvolle Photoresists dar, die eine sehr gute Lichtempfindlichkeit aufweisen. Die durch Säurespaltung aus den bevorzugten erfindungsgemässen Polymeren erhaltenen Verbindungen sind zudem basenlöslich. Dagegen sind die bevorzugten erfindungsgemässen Polymeren gegenüber Basen sehr stabil, so dass im Photoresist eine sehr gute Differenzierung zwischen belichteten und unbelichteten Stellen erhalten wird.

Den erfindungsgemässen Copolymeren, die, gelöst in einem organischen Lösungsmittel, direkt als Photoresist eingesetzt werden können, können gegebenenfalls auch Bindemittel zugesetzt werden.

Die Wahl des Bindemittels erfolgt je nach dem Anwendungsgebiet und den hierfür geforderten Eigenschaften, wie Entwickelbarkeit in wässrigen und wässrig-alkalischen Lösungsmittelsystemen oder Adhäsion auf Substraten.

Geeignete Bindemittel sind beispielsweise Novolake, die sich von einem Aldehyd, vorzugsweise Formaldehyd, Acetaldehyd oder Furfuraldehyd, besonders jedoch von Formaldehyd, und einem Phenol ableiten. Die phenolische Komponente dieser Bindemittel ist vorzugsweise Phenol selbst, oder auch halogeniertes Phenol, beispielsweise substituiert mit ein bis zwei Chloratomen, vorzugsweise p-Chlorphenol, oder sie ist ein durch ein bis zwei $C_1$-$C_9$-Alkylgruppen substituiertes Phenol, beispielsweise o- m- oder p-Kresol, ein Xylenol, p-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Weitere geeignete Bindemittel sind beispielsweise Copolymere von Maleinsäureanhydrid mit Styrol oder Vinylethern oder 1-Alkenen. Ebenfalls als Bindemittel einsetzen lassen sich Homo- und copolymere Acrylate und Methacrylate, z.B. Copolymere aus Methylmethacrylat/Aethylacrylat/Methacrylsäure, Poly(methacrylsäurealkylester) oder Poly(acrylsäurealkylester), wobei Alkyl = $C_1$-$C_{20}$ ist.

Vorzugsweise verwendet man als Bindemittel eine alkalilösliche Substanz, beispielsweise einen Novolak (gegebenenfalls modifiziert, wie oben beschrieben), Poly-(4-hydroxystyrol) oder Poly-(4-hydroxy-3,5-dimethylstyrol), Copolymere von Maleinsäureanhydrid mit Styrol oder Vinylethern oder 1-Alkenen, sowie Copolymere von Estern der Acrylsäure oder der Methacrylsäure mit ethylenisch ungesättigten Säuren, beispielsweise Methacrylsäure oder Acrylsäure.

Die erfindungsgemässen Copolymeren können weitere übliche Zusatzstoffe enthalten, wie z.B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen, in einem Lösungsmittel gelösten Copolymeren eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie Metalle, wie Al, Cu, Ni, Fe, Zn, Mg oder Co, und GaAs, Si oder $SiO_2$, bei denen durch bildmässiges Belichten eine Abbildung aufgebracht werden soll.

Die Wahl des Lösungsmittels und die Konzentration richtet sich hauptsächlich nach der Art der Zusammensetzung und nach dem Beschichtungsverfahren. Die Lösung wird mittels bekannten Beschichtungsverfahren auf ein Substrat gleichförmig aufgebracht, z.B durch Schleudern, Tauchen, Rakelbeschichtung, Vorhanggiessverfahren, Aufpinseln, Sprühen, speziell durch elektrostatisches Sprühen und Reverse-Rollbeschichtung. Es ist auch möglich, die lichtempfindliche Schicht auf einen temporären, flexiblen Träger zu bringen, und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Copolymeren in weiter variablen Schichtdicken eingesetzt werden können. Dieser Schichtdickenbereich umfasst Werte von ca. 0,1 μm bis mehr als 10 μm.

Mögliche Einsatzgebiete der erfindungsgemässen Copolymeren sind die Verwendung als Photoresists für

die Elektronik (Galvanoresist, Aetzresist, Lötstoppresist), die Herstellung von Druckplatten, wie Offsetdruckplatten oder Siebdruckformen, der Einsatz beim Formteilätzen oder der Einsatz als Mikroresist bei der Herstellung integrierter Schaltkreise. Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit Kunststoff beschichtete Papiere; für Offsetdruckformen speziell behandeltes Aluminium, für die Herstellung gedruckter Schaltungen kupferkaschierte Laminate und für die Herstellung von integrierten Schaltkreisen Siliziumwafer. Die Schichtdicken für photographische Materialien und Offsetdruckformen betragen ca. 0,5 μm bis 10 μm; für gedruckte Schaltungen 0,4 bis ca. 2 μm.

Nach dem Beschichten wird das Lösungsmittel in der Regel durch Trocknen entfernt und es resultiert eine Schicht des Photoresists auf dem Träger.

Nach der in üblicher Weise erfolgten bildmässigen Belichtung des Materials werden die belichteten Stellen des Photolackes durch Herauslösen in einem Entwickler entfernt.

Die Wahl des jeweiligen Entwicklers richtet sich nach der Art des Photolackes, insbesondere nach der Natur des verwendeten Bindemittels oder der entstehenden Photolyseprodukte. Der Entwickler kann wässrige Lösungen von Basen umfasssen, denen gegebenenfalls organische Lösungsmittel oder deren Mischungen zugesetzt wurden.

Besonders bevorzugt als Entwickler werden wässrig alkalische Lösungen, wie sie auch für die Entwicklung von Naphthochinondiazidschichten eingesetzt werden. Dazu zählen insbesondere wässrige Lösungen von Alkalimetallsilikaten, -phosphaten, -hydroxiden und -carbonaten. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Der Begriff 'bildmässige' Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteten Substrates bewegt wird, und auf diese Weise ein Bild erzeugt, sowie die Bestrahlung mit computergesteuerten Elektronenstrahlen.

Die Lichtempfindlichkeit der erfindungsgemässen Copolymeren reicht in der Regel vom UV-Gebiet (ca. 200 nm) bis ca. 600 nm und umspannt somit einen sehr breiten Bereich. Als Lichtquellen kommen daher eine grosse Anzahl der verschiedensten Typen zur Anwendung. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen, photographische Flutlichtlampen, Elektronenstrahlen und Röntgenstrahlen. Der Abstand zwischen Lampe und erfindungsgemässem Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser. Bei dieser Art der Belichtung ist eine Photomaske im Kontakt mit der Photopolymerschicht nicht mehr unbedingt nötig; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die Erfindung betrifft daher auch die unter Verwendung der erfindungsgemässen Copolymeren hergestellten Druckformen, gedruckten Schaltungen, integrierten Schaltkreise oder silberlosen photographischen Filme.

Herstellung von 4-Hydroxydiphenylsulfid

173 g (1 Mol) 4-Bromphenol, 184,8 g (1,07 Mol) Thiophenol-Kupfersalz, 80 ml Pyridin und 800 ml Chinolin werden in einem 1,5 Liter-Sulfierkolben mit Rührer vorgelegt. Unter Stickstoff wird bei 210°C 16 Stunden (h) lang gerührt. Die homogene Lösung wird auf etwa 120°C abgekühlt und auf Eis/Salzsäure gegossen. Man gibt 1 Liter Chloroform dazu und rührt das Ganze gut durch.

Die organische Phase wird abgetrennt und einmal mit 10%-iger Salzsäure gewaschen. Die organische Phase wird auf 15%-ige Natronlauge gegossen, gut durchgerührt und die wässrige Phase abgetrennt. Die wässrige Phase wird einmal mit Ether gewaschen.

Die wässrige Phase wird mit konzentrierter Salzsäure sauer gestellt und das Produkt zweimal mit Ether extrahiert. Die Etherphase wird getrocknet, mit Aktivkohle behandelt und dann eingedampft. Die erhaltene Flüssigkeit wird bei 0,01 mbar über eine Vigreuxkolonne destilliert. Der Siedepunkt liegt bei 115°C.

Die erhaltene Substanz wird fest und lässt sich in n-Hexan umkristallisieren. Man erhält farblose Nadeln,

die bei 43,5°C schmelzen. Ausbeute: 52,3 g (26 % der Theorie).

Elementaranalyse (Mikroanalyse):

| | berechnet | gefunden |
|---|---|---|
| C = | 71,26 % | 71,06 % |
| H = | 4,98 % | 5,00 % |
| S = | 15,85 % | 15,90 %. |

Das $^1H$-NMR-Spektrum ($CDCl_3$) stimmt mit der Struktur für 4-Hydroxydiphenylsulfid überein.

Herstellung von 4-Hydroxyphenyldiphenylsulfoniumhexafluoroarsenat

8 g 4-Hydroxydiphenylsulfid (40 mMol) und 18,8 g Diphenyljodoniumhexafluoroarsenat (40 mMol), hergestellt nach der von J.V. Crivello und J.H.W. Lam in J. Org. Chem. 43 (15), 1978, Seiten 3055-58 angegebenen Arbeitsvorschrift, werden zusammen mit 0,5 g Kupfer(II)benzoat in einen 100 ml Rundkolben vorgelegt und unter Stickstoff während 4 h bei 130°C gerührt.

Dann wird auf 20°C abgekühlt und mit Ether versetzt. Ein Festkörper fällt aus, der gründlich mit Ether gewaschen und abgetrennt wird. Der Festkörper wird in Chloroform gelöst und auf Ether getropft. Das erhaltene Oel wird abgetrennt und am Hochvakuum bei 40°C getrocknet. Man erhält ein Pulver. Ausbeute: 9,4 g (50 % der Theorie).

Mikroanalyse:

| | berechnet | gefunden |
|---|---|---|
| C = | 46,17 % | 46,69 % |
| H = | 3,23 % | 3,22 % |
| S = | 6,85 % | 7,23 %. |

Das $^1H$-NMR-Spektrum (Aceton-$d_6$) stimmt mit der Struktur für 4-Hydroxyphenyldiphenylsulfoniumhexafluoroarsenat überein.

Herstellung von 4-Hydroxyphenyldiphenylsulfoniumhexafluoroantimonat

Es wird nach oben angegebener Vorschrift gearbeitet, wobei das eingesetzte Diphenyljodoniumhexafluorantimonat ebenfalls nach der in J. Org. Chem. 43 (15), 1978, Seiten 3055-58 angegebenen Arbeitsvorschrift hergestellt wurde. Ausbeute: 35 % der Theorie.

Das $^1H$-NMR-(Aceton-$d_6$) stimmt mit der Struktur für 4-Hydroxyphenyldiphenylsulfoniumhexafluoroantimonat überein.

Mikroanalyse:

| | berechnet | gefunden |
|---|---|---|
| C = | 41,97 % | 42,47 % |
| H = | 2,94 % | 2,96 % |
| S = | 6,22 % | 6,37 %. |

Herstellung von 4-Hydroxyphenyldiphenylsulfonium-trifluormethansulfonat(-triflat)

Es wird nach oben angegebener Vorschrift gearbeitet, wobei das eingesetzte Diphenyljodoniumtriflat nach der in J. Org. Chem. 43 (15), 1978, Seiten 3055-58 angegebenen Arbeitsvorschrift hergestellt wurde. Ausbeute an Triflat-Salz: 48 % der Theorie.

Dieses Salz lässt sich in n-Butanol umkristallisieren. Schmelzpunkt: 143°C.

Mikroanalyse:

| | berechnet | gefunden |
|---|---|---|
| C = | 53,39 % | 53,40 % |
| H = | 3,30 % | 3,42 % |
| S = | 15,00 % | 14,77 %. |

Das $^1H$-NMR-Spektrum (Aceton-$d_6$) stimmt mit der Struktur für 4-Hydroxyphenyldiphenylsulfoniumtriflat überein.

Herstellung von 4-Vinylbenzoesäuretetrahydropyran-2-ylester

20 g ( 135 mMol) 4-Vinylbenzoesäure und 22,7 g (270 mMol) 3,4-Dihydro-2H-pyran werden in einem 100 ml Rundkolben mit Magnetrührer vorgelegt. Zur Suspension gibt man 4 Tropfen konzentrierter Salzsäure. Unter Stickstoff wird das Reaktionsgemisch bei 40°C gerührt. Nach etwa 40 Minuten erhält man eine klare Lösung, die noch 40 Minuten weitergerührt wird. Die Lösung wird auf eiskalte 2n-Natriumhydroxidlösung gegossen und zweimal mit Diethylether ausgezogen. Die Etherphase wird mit Wasser gewaschen und mit Natriumsulfat getrocknet. Nach dem Abdampfen des Ethers am Rotationsverdampfer wird die erhaltene Flüssigkeit in n-Hexan gelöst und mit Aktivkohle behandelt, filtriert und das Lösungsmittel am Rotationsverdampfer entfernt. Die klare, farblose Müssigkeit wird anschliessend am Hochvakuum getrocknet. Ausbeute: 27,5 g (88 % der Theorie). Die Substanz ist nicht destillierbar.

Elementaranalyse (Mikroanalyse):

| | berechnet | gefunden |
|---|---|---|
| C = | 72,39 % | 72,41 % |
| H = | 6,94 % | 7,23 %. |

Das $^1H$-NMR-Spektrum ($CDCl_3$) stimmt mit der Struktur für 4-Vinylbenzoesäuretetrahydropyran-2-ylester überein.

Herstellung von 4-Tetrahydropyran-2-yloxystyrol

a) In einem 1 Liter-Sulfierkolben mit Rückflusskühler, Stickstoffüberleitung und mechanischem Rührer werden 150 g (0,91 Mol) 4-Hydroxyzimtsäure, 6 g (54,5 mMol) Hydrochinon und 6 g 1,8-Diazobicyclo[5,4,0]-undecen-7 (39,4 mMol) in 300 ml Dimethylsulfoxid (DMSO) gelöst. Die Reaktionsmischung wird während 4 h bei einer Innentemperatur von 130-135°C gerührt, anschliessend auf Eis gegossen und in Ether aufgenommen. Die Etherextrakte werden 5 mal mit 500 ml Wasser gewaschen, mit $MgSO_4$ getrocknet und eingeengt. Umkristallisation aus n-Hexan liefert 62,9 g (57 % der Theorie) 4-Hydroxystyrol mit einem Schmelzpunkt (Smp.) von 67,8°C.

Durch Konzentrieren der Mutterlauge werden weitere 10,25 g (9,6 % der Theorie) eines Produktes mit einem Smp. von 64,2°C erhalten.

Das $^1H$-NMR-Spektrum (DMSO) stimmt mit der Struktur für 4-Hydroxystyrol überein.

b) In einem 250 ml 3-Halsrundkolben mit Magnetrührer und $N_2$-Ueberleitung werden 15 g (0,125 Mol) 4-Hydroxystyrol in 120 ml Ether und 0,4 g p-Toluolsulfonsäure-monohydrat gelöst. Bei 5°C Innentemperatur werden 11,6 g (0,1375 Mol) 3,4-Dihydro-2H-pyran tropfenweise zugegeben. Nach 30 Minuten wird die Kühlung entfernt und die Reaktionsmischung während 20 h bei Raumtemperatur gerührt.

Danach wird mit in wässriger NaOH-Lösung neutralisiert, die Etherphase anschliessend 3 mal mit Wasser gewaschen. Man trocknet über $MgSO_4$ und engt ein, wobei 23,3 g (90 % der Theorie) 4-Tetrahydropyran-2-yloxystyrol als farbloses Oel in dünnschichtchromatographisch-reiner Form zurückbleiben, welches direkt der Polymerisation zugeführt wird.

Das $^1H$-NMR-Spektrum ($CDCl_3$) stimmt mit der Struktur für 4-Tetrahydropyran-2-yloxystyrol überein.

Beispiel 1: Herstellung von 4-Acryloyloxyphenyldiphenylsulfoniumhexafluoroarsenat

9 g ( 19,2 mMol) 4-Hydroxyphenyldiphenylsulfoniumhexafluoroarsenat und 1,9 g (19,2 mMol) Triethylamin werden in 36 ml Methylenchlorid gelöst. Unter Stickstoff wird langsam 1,7 g ( 19,2 mMol) Acrylsäurechlorid zugetropft. Dabei steigt die Temperatur auf etwa 35°C. Die farblose Lösung wird bei 25°C noch etwa $1\frac{1}{2}$ h gerührt.

Man giesst auf Eis und wäscht die organische Phase mehrmals mit Wasser. Die organische Phase wird mit Natriumsulfat getrocknet und eingedampft. Bei 0,01 mbar wird bei 30°C getrocknet. Man erhält 8,1 g (81 % der Theorie) eines Festkörpers.

Elementaranalyse (Mikroanalyse):

| | berechnet | gefunden |
|---|---|---|
| C = | 48,29 % | 48,30 % |
| H = | 3,28 % | 3,28 % |
| S = | 6,14 % | 6,33 % |
| F = | 21,82 % | 21,59 % |
| As = | 14,34 % | 13,5 %. |

Das IR-(KBr-Pille) und $^1$H-NMR-Spektrum (Aceton-$d_6$) stimmen mit der Struktur für 4-Acryloyloxyphenyldiphenylsulfoniumhexafluoroarsenat überein.

Beispiel 2: Herstellung von 4-Acryloyloxyphenyldiphenylsulfoniumhexafluoroantimonat

Analog der im Beispiel 1 angegebenen Vorschrift wird 4-Hydroxyphenyldiphenylsulfoniumhexafluoroantimonat mit Acrylsäurechlorid umgesetzt. Ausbeute an Antimonatsalz: 51 % der Theorie.

Mikroanalyse:

| | berechnet | gefunden |
|---|---|---|
| C = | 44,32 % | 44,95 % |
| H = | 3,01 % | 3,00 % |
| S = | 5,63 % | 5,74 %. |

Das IR-(KBr) und $^1$H-NMR-Spektrum (Aceton-$d_6$) stimmen mit der Struktur für 4-Acryloyloxyphenyldiphenylsulfoniumhexafluoroantimonat überein.

Beispiel 3: Herstellung von 4-Acryloyloxyphenyldiphenylsulfoniumtriflat

Analog der im Beispiel 1 angegebenen Vorschrift wird 4-Hydroxyphenyldiphenylsulfoniumtriflat mit Acrylsäurechlorid umgesetzt. Ausbeute an Triflatsalz: 45 % der Theorie.

Mikroanalyse:

| | berechnet | gefunden |
|---|---|---|
| C = | 54,77 % | 53,91 % |
| H = | 3,55 % | 3,67 % |
| S = | 13,29 % | 12,99 %. |

Das IR-(KBr) und $^1$H-NMR-Spektrum (Aceton-$\alpha_6$) stimmen mit der Struktur für 4-Acryloyloxyphenyldiphenylsulfoniumtriflat überein.

Beispiel 4: Herstellung von4-Allyloxyphenyldiphenylsulfoniumhexafluoroarsenat

10 g (21,4 mMol) 4-Hydroxyphenyldiphenylsulfoniumhexafluoroarsenat, 38,8 g (3,20 mMol) 3-Brom-1-propen und 15 g Kaliumcarbonat werden in 500 ml Aceton vorgelegt. Unter Stickstoff wird das Gemisch bei 65°C kräftig gerührt. Nach 18 Stunden wird der Festkörper abgetrennt und die Lösung am Rotationsverdampfer vom Lösungsmittel befreit. Der Rückstand wird in Methylenchlorid gelöst, mit Aktivkohle behandelt, filtriert und auf Diethylether getropft. Es fällt ein farbloses Oel aus. Dieses wird in Ethanol aufgenommen, mit Aktivkohle behandelt, filtriert und aus Ethanol auskristallisiert.

Es werden farblose Kristalle erhalten. Ausbeute: 6,6 g (62 % der Theorie).
Schmelzpunkt: 85,6°C.

Mikroanalyse:

| | berechnet | gefunden |
|---|---|---|
| C = | 49,66 % | 49,69 % |
| H = | 3,77 % | 3,65 % |
| S = | 6,31 % | 6,27 % |
| F = | 22,42 % | 22,51 % |
| As= | 14,74 % | 14,7 %. |

Das $^1H$-NMR-Spektrum in $CDCl_3$ stimmt mit der Struktur für 4-Allyloxyphenyldiphenylsulfoniumhexafluorarsenat überein.

Beispiel 5: Copolymer I

8 g (34,4 mMol) 4-Vinylbenzoesäuretetrahydropyran-2-ylester, 0,42 g (0,8 mMol) 4-Acryloyloxyphenyldiphenylsulfoniumhexafluoroarsenat (APDSH), 113 mg (0,69 mMol) $\alpha,\alpha'$-Azoisobutyronitril (AIBN) und 32 ml trockenes Tetrahydrofuran (THF) werden in einer 50 ml Ampulle vorgelegt. Bei -78°C wird die Lösung vom Sauerstoff befreit und die Ampulle unter Vakuum zugeschmolzen. Bei 60°C wird 13 h lang polymerisiert. Die viskose Lösung wird auf Methanol getropft wobei das Polymer ausfällt. Das getrocknete Polymer wird in THF gelöst und erneut in n-Hexan gefällt. Das weisse Polymerpulver wird am Hochvakuum bei 40°C getrocknet. Ausbeute: 5,7 g (68 % der Theorie).

Gelpermeationschromatographie (GPC) in THF:
Mw: 11'000 Mn: 4'000 Mw/Mn 2,7.

Mikroanalyse:

| | berechnet | gefunden |
|---|---|---|
| C = | 69,9 % | 70,7 % |
| H = | 6,5 % | 6,7 % |
| S = | 0,6 % | 0,5 %. |

Beispiel 6: Copolymer II

In einem 25 ml-Rundkolben werden in 8 g frisch destilliertem THF 1,9 g 4-Tetrahydropyran-2-yloxystyrol (TPOS) und 0,1 g APDSH mit 30 mg $\alpha,\alpha'$-Azoisobutyronitril versetzt. Man lässt bei 70°C während 20 h reagieren. Danach tropft man die Lösung zur 10-fachen Menge n-Hexan. Der resultierende weisse Niederschlag wird auf der Glasnutsche vom Fällmittel befreit und im Vakuum getrocknet. Ausbeute: 0,48 g eines weissen Pulvers.

Elementaranalyse:

| | berechnet | gefunden |
|---|---|---|
| C = | 73,1 % | 72,25 % |
| H = | 7,35 % | 7,38 % |
| S = | 0,73 % | 1,26 %. |

GPC: Mn = 3979
Mw = 6813
Mw/Mn = 1,71.

Beispiel 7: Copolymer III

In einer im Vakuum zugeschmolzenen Glasampulle werden in 8 g frisch destilliertem THF 19 g TPOS, 0,1 g APDSH und 30 mg α,α'-Azoisobutyronitril während 20 h auf 70°C erhitzt. Danach wird in der 10-fachen Menge n-Hexan ausgefällt, der resultierende weisse Niederschlag auf der Glasnutsche vom Fällungsmittel befreit und im Vakuum getrocknet. Ausbeute: 0,90 g.

Elementaranalyse:

| | berechnet | gefunden |
|---|---|---|
| C = | 73,1 % | 73,1 % |
| H = | 7,3 % | 7,41 % |
| S = | 0,73 % | 0,93 %. |

GPC: Mn = 3116
Mw = 9635
Mw/Mn = 3,09.

Beispiel 8: Copolymer IV

In einer Ampulle werden 5,9 g (28,9 mMol) 4-Allyloxyphenyldiphenylsulfoniumhexafluoroarsenat und 115 mg (0,7 mMol) AIBN in 30 ml THF gelöst. Die Lösung wird entgast, und die Ampulle wird unter Hochvakuum zugeschmolzen. Der Inhalt wird bei 65°C während 17 Stunden polymerisiert. Dann wird das Polymer in Methanol ausgefällt. Erneutes Lösen in THF und Fällen in n-Hexan liefert nach dem Trocknen ein weisses Polymer. Ausbeute: 2,9 g (43 % der Theorie).

Elementaranalyse:

| | gefunden |
|---|---|
| C = | 75,70 % |
| H = | 7,88 % |
| S = | 0,21 %. |

Gemäss der Elementaranalyse wurden 1,5 Gewichtsprozente des Oniumsalzes einpolymerisiert.

Gelpermeationschromatographie (GPC) in THF:

Mw = 14800 Mn = 9000 Mw/Mn = 1,6

Temperaturgravimetrie (TGA): Abspaltung der Dihydropyrangruppe bei 233°C (onset).

Anwendungsbeispiele

Beispiel A

1,4 g Copolymer I gemäss Beispiel 5 wird in 7 g Cyclopentanon gelöst und durch ein 0,5 Mikron Filter filtriert. Diese Lösung wird auf eine Siliziumscheibe aufgetragen und mittels Spinnbeschichtung wird ein homogener Polymerfilm erzeugt. Der Film wird bei 90°C während 2 Minuten getrocknet. Die Filmdicke beträgt 1 Mikron. Nach Belichtung durch eine Maske mit Licht der Wellenlänge von 290 nm ± 20 nm (5-8 mJ/cm$^2$) wird der Resist 1 Minute lang auf einer Heizplatte von 110°C beheizt. Der Resist wird nun in 0,5%-iger wässrigem Natriumbicarbonat entwickelt. Man erhält ein positives Bild. Strukturen im Submikronbereich sind aufgelöst.

Beispiel B

1 Teil des Copolymers II wird in 3 Teilen Cyclohexanon gelöst. Die filtrierte Lösung wird bei 3500 Umdrehungen/min. mittels Drehschleudern auf eine Siliziumscheibe aufgetragen und danach bei 120°C während 120 Sekunden getrocknet. Der resultierende Resistfilm weist eine Dicke von 0,8 gm auf. Die Bestrahlung geschieht mittels Hg-Dampflampe mit Engbandinterferenzfilter (10 nm Bandbreite, 254 nm) durch eine Quarzmaske, deren kleinste Strukturen Dimensionen von 0,5 µm aufweisen; Dosis: 1-20 mJ/cm$^2$. Unmittelbar nach der Belichtung wird der Wafer bei 90°C während 90 Sekunden getempert und direkt anschliessend entwickelt.

| | |
|---|---|
| Entwickler: | 2n wässrige NaOH-Lösung oder Shipley®MF312 |
| Entwicklungszeit: | 30- 120 Sekunden |
| Resultate: | 0,5 µm Strukturen aufgelöst, vertikale Wandprofile, Kontrast $\gamma$ = 4,0, thermische Beständigkeit > 130°C. |

Beispiel C

Ein Teil des Copolymers IV wird in 5 Teilen Cyclopentanon gelöst. Die Lösung wird durch ein 0,5 Mikron Filter auf eine Siliziumscheibe aufgebracht. Durch Spin-Coating bei 3500 Umdrehungen/Min. wird ein dünner Film erzeugt, der bei 120°C während 60 Sekunden getrocknet wird. Der Resist weist eine Schichtdicke von 1 Mikron auf. Der Resist wird durch eine Quarzmaske und ein 254 nm Engbandfilter hindurch mit einer Dosis von 10 mJ/cm$^2$ belichtet. Nach der Belichtung wird der Resist bei 90°C während 100 Sekunden erwärmt und in der Entwicklungslösung Shipley® MF312 während etwa 60 Sekunden entwickelt.

Es lassen sich Submikronstrukturen mit vertikalen Wandprofilen auflösen.

## Patentansprüche

1. Olefinisch ungesättigte Oniumsalze der Formeln I und II

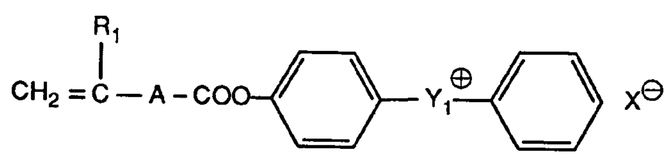

(I)

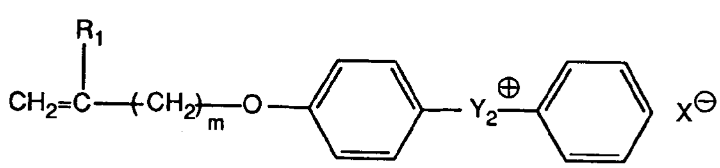

(II),

worin $R_1$ ein Wasserstoffatom oder Methyl und A eine direkte Bindung, Phenylen oder einen der folgenden Reste

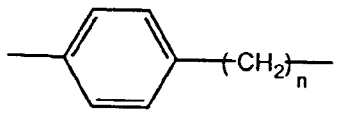 oder $-(CH_2)_o-$

bedeuten, wobei n und o unabhängig voneinander je für eine Zahl von 1 bis 10 stehen, $Y_1$ für J oder S-$R_2$ und $Y_2$ für S-$R_2$ stehen, wobei $R_2$ ein $C_1$-$C_4$-Alkyl, unsubstituiertes oder $C_1$-$C_4$-alkylsubstituiertes Phenyl bedeutet, m für Null oder eine Zahl von 1 bis 10 und X für $BF_4$, $PF_6$, $AsF_6$, $SbF_6$, $SbCl_6$, $FeCl_4$, $SnF_6$, $BiF_6$, $CF_3SO_3$ oder $SbF_5OH$ stehen.

**2.** Oniumsalze der Formeln I und II gemäss Anspruch 1, worin $R_1$ ein Wasserstoffatom oder Methyl und A eine direkte Bindung, Phenylen oder Benzylen bedeuten, $Y_1$ und $Y_2$ je für S-$R_2$ stehen, wobei $R_2$ ein unsubstituiertes oder methylsubstituiertes Phenyl bedeutet, m für Null oder eine Zahl von 1 bis 4 und X für $AsF_6$, $SbF_6$ oder $CF_3SO_3$ stehen.

**3.** Oniumsalze der Formeln I und II gemäss Anspruch 1, worin $R_1$ ein Wasserstoffatom und A eine direkte Bindung bedeuten, $Y_1$ und $Y_2$ je für

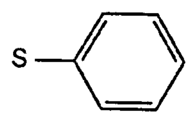

stehen, m Null bedeutet und X für $AsF_6$, $SbF_6$ oder $CF_3SO_3$ steht.

**4.** 4-Acryloyloxyphenyldiphenylsulfoniumhexafluoroarsenat, 4-Acryloyloxyphenyldiphenylsulfoniumhexafluoroantimonat, 4-Acryloyloxyphenyldiphenylsulfoniumtriflat und Allyloxyphenyldiphenylsulfoniumhexafluoroarsenat als Verbindungen der Formel I oder II gemäss Anspruch 1.

**5.** Verfahren zur Herstellung von olefinisch ungesättigten Oniumsalzen der Formeln I und II gemäss Anspruch 1, dadurch gekennzeichnet, dass man 1 Mol eines hydroxysubstituierten Oniumsalzes der Formel III

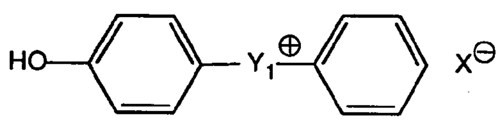 (III),

worin $Y_1$ und X die gleiche Bedeutung wie in Formel I haben, mit 1 Mol eines Säurehalogenids der Formel IV

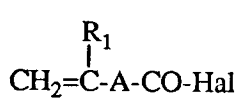 (IV),

worin $R_1$ und A die gleiche Bedeutung wie in Formel I haben und Hal für ein Chlor-, Brom- oder Jodatom steht, in Gegenwart eines tertiären Amins zu Verbindungen der Formel I umsetzt oder, indem man 1 Mol eines hydroxysubstituierten Oniumsalzes der Formel V

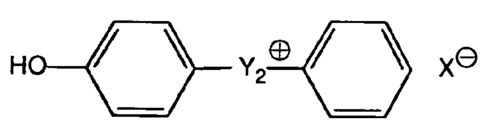 (V),

worin $Y_2$ und X die gleiche Bedeutung wie in Formel II haben, mit 1 Mol einer Halogenverbindung der Formel VI

$$CH_2=CH-(CH_2)_m-Hal \qquad (VI),$$

worin m die gleiche Bedeutung wie in Formel II hat und Hal für ein Chlor-, Brom- oder Jodatom steht, in Gegenwart eines tertiären Amins zu Verbindungen der Formel II umsetzt.

6. Strahlungsempfindliche Copolymere mit einem Molekulargewicht (Mw) von $10^3$ bis $10^6$, gemessen mittels Gelpermeationschromatographie, enthaltend, bezogen auf die Gesamtsumme der im Copolymer vorhandenen Strukturelemente,

(a) 1 bis 20 Mol% des wiederkehrenden Strukturelementes der Formel VII oder VIII

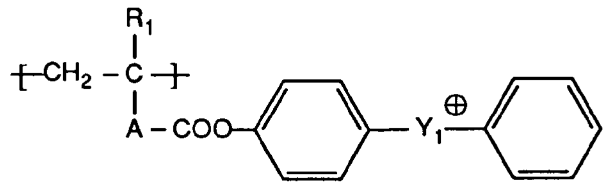

(VII) oder

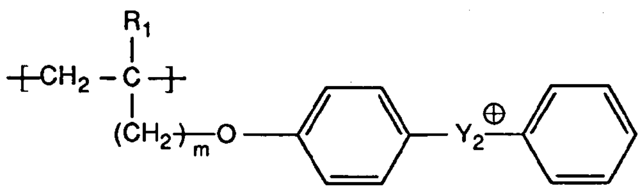

(VIII),

worin $R_1$, A, $Y_1$, $Y_2$ und m die gleiche Bedeutung wie in Formel I bzw. II haben,

(b) 80 bis 99 Mol% eines wiederkehrenden Strukturelementes der Formel IX

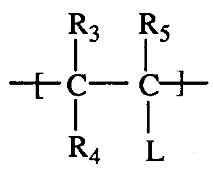

(IX),

worin $R_3$ und $R_4$ unabhängig voneinander je ein Wasserstoffatom, $C_1$-$C_4$-Alkyl oder Phenyl, $R_5$ ein Wasserstoffatom, Methyl oder ein Halogenatom und L einen eine säurelabile Gruppe enthaltenen Rest bedeuten, und

(c) 0 bis 50 Mol% eines wiederkehrenden Strukturelementes der Formel X

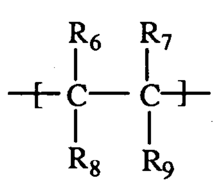

(X),

worin $R_6$ und $R_7$ unabhängig voneinander je ein Wasserstoffatom, unsubstituiertes oder durch Halogenatome, Cyano- oder Nitrogruppen substituiertes $C_1$-$C_4$-Alkyl oder ein unsubstituiertes oder durch Halogenatome, $C_1$-$C_4$-Alkoxy-, Hydroxy-, Cyano- oder Nitrogruppen substituiertes Phenyl oder Naphthyl bedeuten, und $R_8$ und $R_9$ unabhängig voneinander je ein Wasserstoff- oder Halogenatom, unsubstituiertes oder durch Halogenatome, Cyano- oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste $-OR_{10}$, $-COOR_{11}$ oder $-COR_{12}$ bedeuten, wobei $R_{10}$ und $R_{11}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-$C_4$-Alkyl oder $C_1$-$C_4$-Alkoxy substituiertes Phenyl oder Naphthyl bedeuten und $R_{12}$ die gleiche

Bedeutung wie $R_{10}$ hat und ausserdem für den Rest

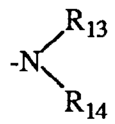

steht, worin $R_{13}$ und $R_{14}$ unabhängig voneinander die gleiche Bedeutung wie $R_{10}$ haben, oder worin $R_8$ und $R_9$, wenn $R_6$ und $R_7$ für ein Wasserstoffatom stehen, zusammen für den Rest

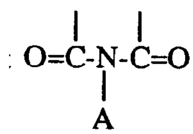

stehen, worin A ein Wasserstoffatom, ein unsubstituiertes oder hydroxysubstituiertes Phenyl oder Benzyl bedeutet, wobei die Summe der Mol% aus (a) und (b) 100 Mol% beträgt und die Mol% von (c) sich auf diese Summe beziehen.

**7.** Copolymere gemäss Anspruch 6, enthaltend (a) 5 bis 20 Mol% des wiederkehrenden Strukturelementes der Formel VII oder VIII, (b) 80 bis 95 Mol% des wiederkehrenden Strukturelementes der Formel IX und (c) 0 bis 30 Mol% eines wiederkehrenden Strukturelementes der Formel X.

**8.** Copolymere gemäss Anspruch 6, enthaltend als (a) das wiederkehrende Strukturelement der Formel VII, worin $R_1$ ein Wasserstoffatom und A eine direkte Bindung bedeuten, $Y_1$ und $Y_2$ je für

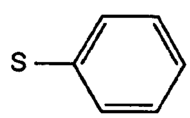

stehen, m Null bedeutet und X für $AsF_6$, $SbF_6$ oder $CF_3SO_3$ steht.

**9.** Copolymere gemäss Anspruch 6, enthaltend als (b) das wiederkehrende Strukturelement der Formel IX, worin L für einen Rest der Formel XI steht

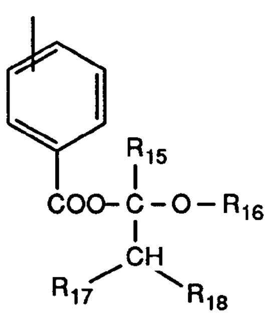

(XI),

worin $R_{15}$ ein Wasserstoffatom oder Methyl, $R_{16}$ ein $C_1$-$C_4$-Alkyl oder $C_6$-$C_{12}$-Aryl und $R_{17}$ und $R_{18}$ unabhängig voneinander je ein Wasserstoffatom, ein $C_1$-$C_4$-Alkyl oder $C_6$-$C_{12}$-Aryl bedeuten, wobei $R_{16}$ und $R_{18}$ zusammen auch einen unsubstituierten oder einen $C_1$-$C_4$-alkyl-, $C_1$-$C_4$-alkoxy-, $C_6$-$C_{12}$-aryl- oder $C_6$-$C_{12}$-aryloxysubstituierten Ethylen-, Propylen- oder Butylenrest bedeuten können.

**10.** Copolymere gemäss Anspruch 6, enthaltend als (b) das wiederkehrende Strukturelement der Formel IX, worin L für einen Rest der Formel XII oder XII steht

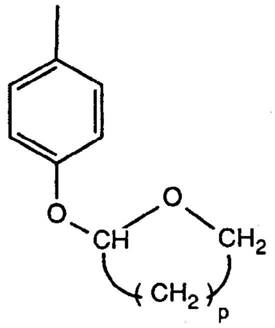 (XII) 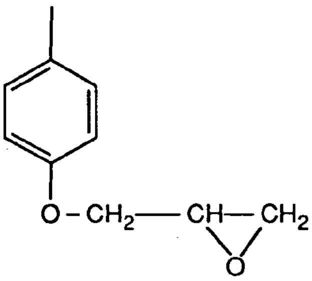 (XIII),

worin p für 2, 3 oder 4 steht.

**11.** Copolymere gemäss Anspruch 6, enthaltend als (b) das wiederkehrende Strukturelement der Formel IX, worin L für einen Rest der Formel XIV steht

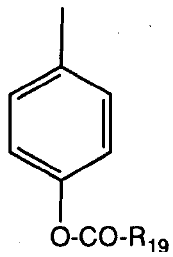

(XIV),

worin $R_{19}$ für $C_1$-$C_4$-Alkyl, $C_1$-$C_4$-Alkoxy oder Oxyalkylen-Si-$(CH_3)_3$ steht.

**12.** Die unter Verwendung der Copolymeren gemäss Anspruch 6 hergestellten Druckformen, gedruckten Schaltungen, integrierten Schaltkreise oder silberlosen photographischen Filme.

Europäisches Patentamt

# EUROPÄISCHER RECHERCHENBERICHT

Nummer der Anmeldung: EP 91810657.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| D,A | DE - A1 - 3 902 114 (BASF) * Ansprüche 1,2,7,8 * -- | 1,5,6,12 | C 07 C 381/12<br>G 03 F 7/027 |
| P,A | EP - A2 -0 406 567 (MITSUBISHI GAS CHEMICAL) * Zusammenfassung * -- | 1 | |
| A | US - A - 3 494 965 (GIFFIN D. JONES et al.) * Zusammenfassung * -- | 1,6 | |
| A | EP - A1 - 0 379 464 (CIBA-GEIGY) * Zusammenfassung * -- | 1,6 | |
| A | US - A - 4 069 054 (GEORGE H. SMITH) * Anspruch 1 * ---- | 1,6 | |

RECHERCHIERTE SACHGEBIETE (Int. Cl.⁵)

C 07 C 381/00
G 03 F 7/00
C 08 F 20/00

Der vorliegende Recherchenbericht wurde für alle Patentansprüche erstellt.

| Recherchenort | Abschlußdatum der Recherche | Prüfer |
|---|---|---|
| WIEN | 07-11-1991 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82